# EUROPEAN PATENT APPLICATION

(11) **EP 1 126 035 A1**
(43) Date of publication of application: **22.08.2001**
(21) Application number: 99952794.8
(22) Date of filing: 28.10.1999
(51) Int. Cl.: C12Q 1/68, C12N 15/12, C12M 1/00, G01N 33/566, G01N 33/53

(54) **METHOD FOR DETECTING GENE AFFECTED BY ENDOCRINE DISRUPTOR**

(30) Priority: 30.10.1998 JP 31028598
(71) Applicant: Takara Shuzo Co, Ltd., Kyoto-shi, Kyoto 612-8061 (JP)
(72) Inventor: KONDO, Akihiro, 2-chome Kusatsu-shi Shiga 525-0025 (JP); SAGAWA, Hiroaki, Kusatsu-shi, Shiga 525-0025 (JP); MINENO, Junichi, Uji-shi, Kyoto 611-0002 (JP); KIMIZUKA, Fusao, Omihachiman-shi, Shiga 523-0056 (JP); KATO, Ikunoshin, Uji-shi, Kyoto 611-0028 (JP)
(74) Representative: Grund, Martin, Dr.
(86) International application number: JP9905964
(87) International publication number: WO0026404

(57) **Abstract**

A method for detecting a gene affected by an endocrine disruptor characterized by comprising preparing a nucleic acid sample containing mRNAs originating in cells, tissues or organisms, which have been brought into contact with a sample containing the endocrine disruptor, or cDNAs thereof; hybridizing the nucleic acid sample with DNA alleys wherein genes which might be affected by the endocrine disruptor or DNA fragments originating in these genes have been fixed; and then comparing the thus obtained results with the results obtained by using another nucleic acid sample originating in a comparative sample to thereby select the gene affected by the endocrine disruptor.

## Description

### Technical Field

The present invention relates to methods for detecting an endocrine disruptor which influences homeostasis of a living body, and a gene influenced by said substance.

### Background Art

Endocrine disruptors (often referred to as environmental hormones) collectively refer to chemical substances released in environment for which hormone-like activities or anti-hormone activities have been found. Altered reproductive potential (in particular, conversion of male into female), decreased reproductive potential, decreased hatchability, decreased survival rate of offspring, abnormal reproductive behavior and the like have been reported to be resulted from the influences of endocrine disruptors on the ecosystem of wild animals. Decreased number of sperms, endometriosis, infertility, ovarian cancer, uterine cancer, prostatic cancer and the like have been suspected to be resulted from the influences of endocrine disruptors on human health, although they have not been demonstrated.

Substances (or groups of substances) that are considered to cause endocrine disruption are reported in the interim report (July, 1997) by "Exogenous Endocrine Disrupting Chemical Task Force" of Environment Agency. However, it is considered that the types of such substances would be further increased in the course of research and study in the future.

Known methods for determining endocrine disruptors are classified into two groups, i.e., in vitro methods and in vivo methods. Examples of the methods in the former group include a method in which a binding activity to estrogen receptor or androgen receptor is measured, and a method in which an activity of inhibiting a hormone synthesis enzyme system is measured. Examples of the methods in the latter group include a method in which production of various hormones and abnormal tissue formation in rats at different postnatal days are measured, a method in which abnormal metamorphosis in a frog is measured, and a method in which abnormal maturation in a fish is measured (Analytical Chemistry, 70(15):528A-532A (1998)).

However, it has not been clearly demonstrated to date whether or not the substances that are suspected to be endocrine disruptors with attention actually cause endocrine disruption. Furthermore, if they cause endocrine disruption, the mechanism through which they influence as well as the amount and the length of the period of intake that might be risky have not been clearly demonstrated.

For example, the current binding test to a hormone receptor is necessary and important as a primary screening. However, the results obtained by this method do not guarantee the identity as an endocrine disruptor. Specifically, estradiol (a naturally occurring female hormone), diethylstilbestrol (a synthetic female hormone), isoflavone (a component contained in pulses which is harmless to humans) and bisphenol-A (a substance suspected to be an endocrine disruptor) bind to estrogen receptor, although the EC₅₀ values for these substances are different each other. Thus, the degree of endocrine disrupting activity of each substance cannot be determined according to this assay method. Similarly, the activity cannot be determined according to any of the conventional methods including an assay system in which a yeast or a cultured cell is used, and a system in which uterine of a mouse is weighed.

In other words, the current methods in which a binding activity to a hormone receptor or an activity of inhibiting hormone synthesis enzyme system is measured in vitro meet a necessary condition as a method for measuring an endocrine disruptor. However, they never meet a sufficient condition. Furthermore, methods in which influences on the growth or morphogenesis of a rat, a frog, a fish or the like are determined in vivo are less sensitive and complicated, and require a long period of time for operating a large number of samples.

In addition, although the conventional analysis methods as described above may be used to analyze the relationship between a potential endocrine disruptor and a receptor, they cannot be used to analyze the downstream signal transduction system.

As described above, it is necessary for solving problems of environmental hormones to identify endocrine disruptors and to determine the influences by the substances on the endocrine systems. Thus, methods for analyzing which signal transduction pathway is influenced by an endocrine disruptor and which substance causes endocrine disruption have been desired.

### Objects of Invention

The main object of the present invention is to provide (1) a method for detecting a gene that is influenced by an endocrine disruptor; (2) a method for detecting a gene that is influenced by an endocrine disruptor which comprises measuring the expression of the gene detected by said method; (3) a DNA array onto which a gene that is influenced by an endocrine disruptor or a DNA fragment derived from the gene is immobilized; and (4) a method for detecting a substance that potentially causes endocrine disruption.

### Summary of Invention

As a result of intensive studies, the present inventors have constructed a method for detecting many types of genes that are influenced by endocrine disruptors rapidly, with high sensitivity and simultaneously. The present inventors have found a method for detecting endocrine disruptors using a DNA array onto which said genes or fragments thereof are immobilized. Furthermore, the present inventors have constructed a. method for detecting a substance that potentially causes endocrine disruption. Thus, the present invention has been completed.

In summary, the present invention relates to:
[1] a method for detecting a gene that is influenced by an endocrine disruptor, characterized in which the method comprises:
   preparing a nucleic acid sample containing mRNAs, or cDNAs therefor, derived from a cell, a tissue or an organism which has been exposed to a sample containing an endocrine disruptor;
   hybridizing the nucleic acid sample with a DNA array onto which genes which are potentially influenced by the endocrine disruptor or DNA fragments derived from the genes which are potentially influenced by the endocrine disruptor are immobilized; and
   selecting a gene that is influenced by the endocrine disruptor by comparing the results with results for a nucleic acid sample prepared using a control sample;
[2] the method according to [1] above, wherein a gene selected from the group consisting of:
   (1) a gene for a nuclear receptor or a gene related to nuclear receptor transcriptional coupling;
   (2) a gene related to kinase-type signal transduction;
   (3) a gene related to gonad differentiation;
   (4) a gene for or related to a receptor-type kinase;
   (5) a gene for or related to an intermediate filament marker;
   (6) a gene related to cell cycle or growth regulation;
   (7) an oncogene, a gene related to an oncogene or a gene related to tumor suppression;
   (8) a gene related to apoptosis;
   (9) a gene related to damage response, repair or recombination of DNA;
   (10) a gene for or related to a receptor;
   (11) a gene related to cell death or differentiation regulation;
   (12) a gene related to adhesion, motility or invasion of cell;
   (13) a gene related to angiogenesis promotion;
   (14) a gene related to cellular invasion;
   (15) a gene related to cell-cell interaction;
   (16) a gene for or related to a Rho family, GTPase or a regulator therefor; and
   (17) a gene for or related to a growth factor or a cytokine,
   or a DNA fragment derived from the gene is used;
[3] a method for detecting an endocrine disruptor, characterized in which the method comprises measuring the expression of the gene detected by the method according to [1] or [2] above;
[4] the method according to [3], wherein the endocrine disruptor is selected from ones classified into:
   (1) dioxins;
   (2) organochlorine compounds;
   (3) phenols;
   (4) phthalate esters;
   (5) aromatic hydrocarbons;
   (6) pesticides;
   (7) organotin compounds;
   (8) estrogens; or
   (9) mirex, toxaphene, aldicarb or kepone;
[5] a method for detecting a substance that potentially causes endocrine disruption, characterized in which the method comprises:
   preparing a nucleic acid sample containing mRNAs, or cDNAs therefor, derived from a cell, a tissue or an organism which has been exposed to a sample that is suspected to contain a substance that potentially causes endocrine disruption;
   hybridizing the nucleic acid sample with a DNA array onto which genes which are influenced by an endocrine disruptor or DNA fragments derived from the genes which are influenced by the endocrine disruptor are immobilized; and
   detecting a substance that potentially causes endocrine disruption by comparing the results with results for a nucleic acid sample prepared using a control sample;
[6] the method according to [5] above, wherein the substance that potentially causes endocrine disruption is classified into:
   (1) dioxins;
   (2) organochlorine compounds;
   (3) phenols;
   (4) phthalate esters;
   (5) aromatic hydrocarbons;
   (6) pesticides;
   (7) organotin compounds;
   (8) estrogens; or
   (9) mirex, toxaphene, aldicarb or kepone;
[7] a DNA array for detecting a gene that is influenced by an endocrine disruptor, onto which a gene that is influenced by an endocrine disruptor or a gene that is potentially influenced by an endocrine disruptor, or a DNA fragment derived from the gene is immobilized;
[8] the DNA array according to [7] above, onto which a gene selected from the group consisting of:
   (1) a gene for a nuclear receptor or a gene related to nuclear receptor transcriptional coupling;
   (2) a gene related to kinase-type signal transduction;
   (3) a gene related to gonad differentiation;
   (4) a gene for or related to a receptor-type kinase;
   (5) a gene for or related to an intermediate filament marker;
   (6) a gene related to cell cycle or growth regulation;
   (7) an oncogene, a gene related to an oncogene or a gene related to tumor suppression;
   (8) a gene related to apoptosis;
   (9) a gene related to damage response, repair or recombination of DNA;
   (10) a gene for or related to a receptor;
   (11) a gene related to cell death or differentiation regulation;
   (12) a gene related to adhesion, motility or invasion of cell;
   (13) a gene related to angiogenesis promotion;
   (14) a gene related to cellular invasion;
   (15) a gene related to cell-cell interaction;
   (16) a gene for or related to a Rho family, GTPase or a regulator therefor; and
   (17) a gene for or related to a growth factor or a cytokine,
   or a DNA fragment derived from the gene is immobilized; and
[9] the DNA array according to [7] or [8] above, wherein the gene or the DNA fragment derived from the gene is immobilized onto a slide glass.

### Detailed Description of the Invention

### (1) The method for detecting a gene that is influenced by an endocrine disruptor of the present invention

As used herein, an endocrine disruptor (also referred to as an exogenous endocrine disruptor or an environmental hormone) means an exogenous substance which, when incorporated into a living body of an animal, influences a normal activity of a hormone naturally exerted in the living body. The endocrine disruptors include ones that maintain, promote or suppress a normal activity of a hormone. Substances that potentially influence a normal activity of a hormone are also included within the definition.

Currently, about 70 substances (or groups of substances) are suspected to have endocrine disrupting activities. These substances are classified in the materials for the abstract of 24th Meeting of the Japan Society for Environmental Chemistry (1998) as follows based on the methods for analyzing the corresponding substances:
(1) Category 1: organochlorine compounds (e.g., general organochlorine compounds, polychlorinated biphenyl (PCB));
(2) Category 2: phenols (e.g., general phenols, bisphenol-A, 2,4-dichlorophenol, pentachlorophenol), (3) Category 3: phthalate esters (e.g., general phthalate esters); (4) Category 4: aromatic hydrocarbons (e.g., benzo(a)pyrene, di-2-ethylhexyl adipate (DEHA), benzophenone, 4-nitrotoluene, stylene dimer and trimer, 1,2-dibromo-3-chloropropane, styrene, n-butylbenzene); (5) Category 5: pesticides (e.g., general pesticides, 2,4,5-T (trichlorophenoxyacetic acid), 2,4-D (dichlorophenoxyacetic acid), benomyl, amitrole, methomyl); (6) Category 6: organotin compounds; (7) Category 7: estrogens; dioxins which are not classified into the above-mentioned seven categories; and substances which are excluded from the categories, i.e., mirex, toxaphene, aldicarb and kepone (chlordecone). These categories and the substances are shown in Table 1.

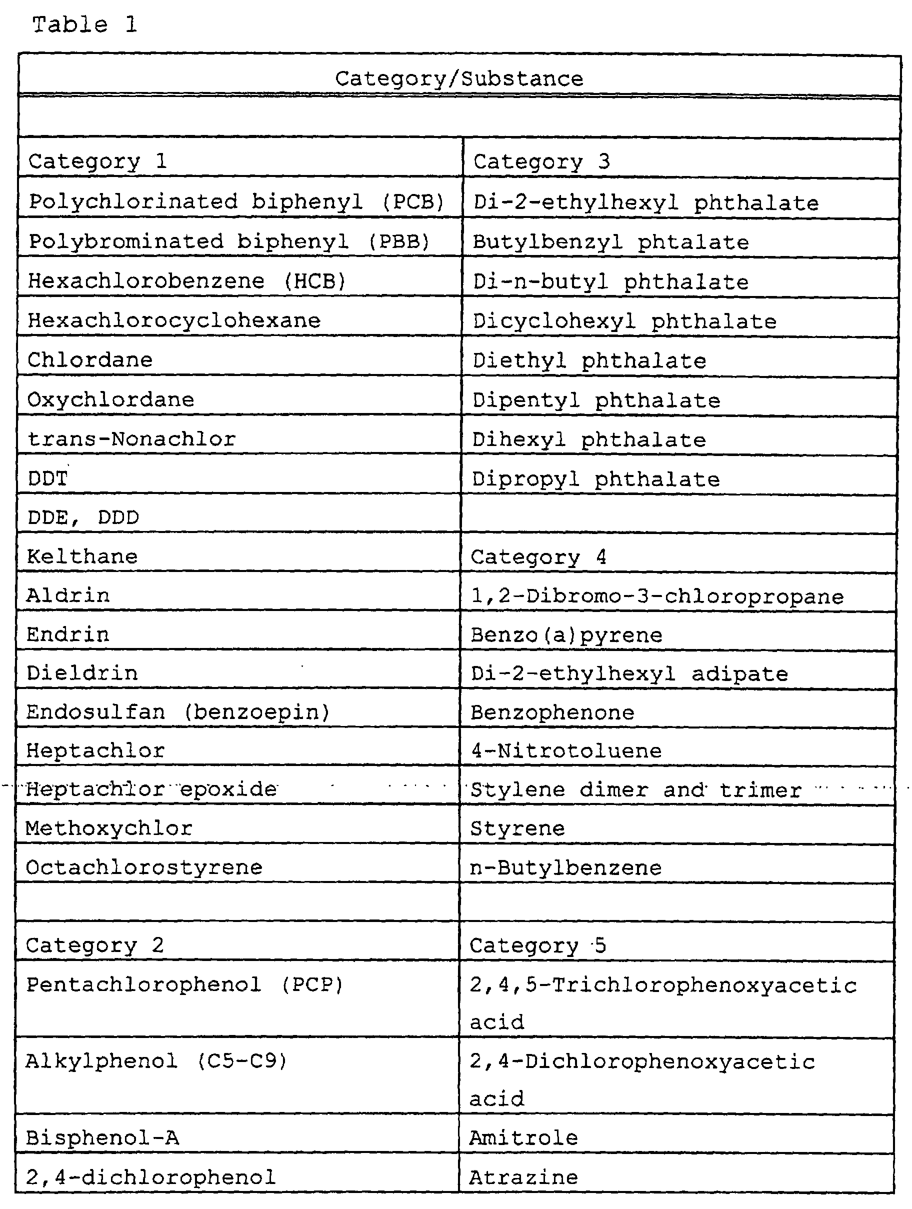

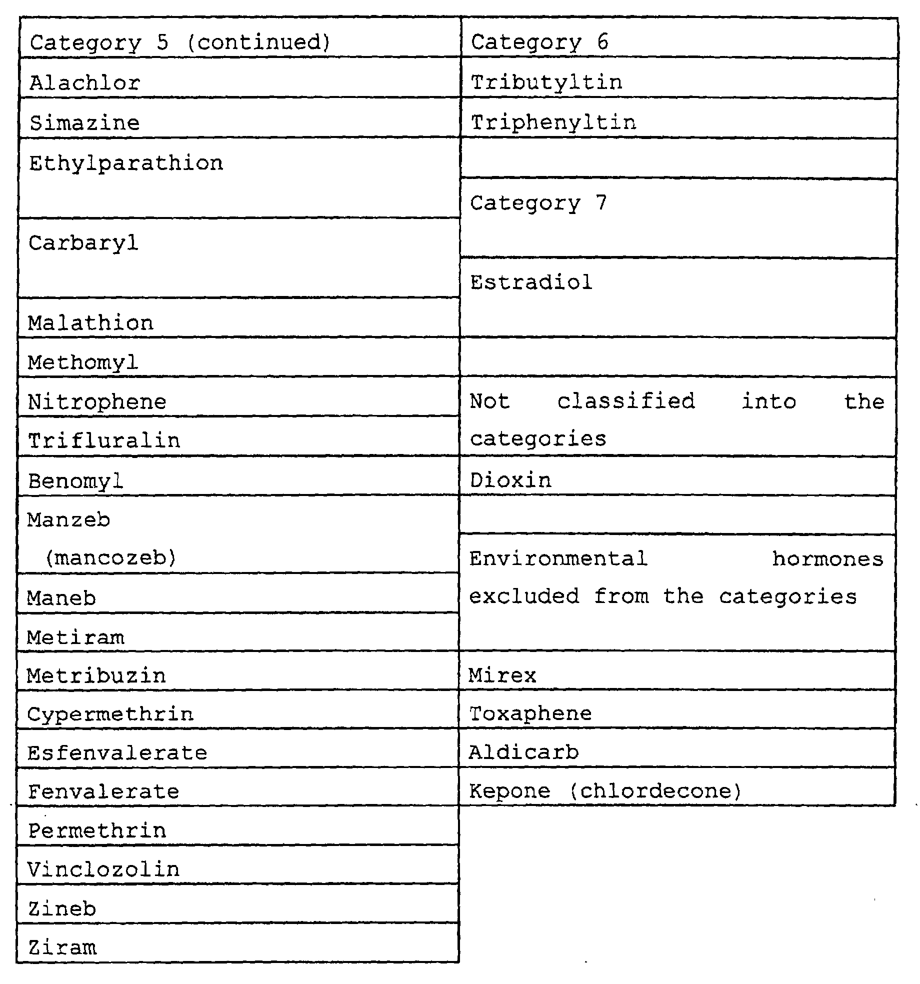

The endocrine disruptors are not limited to those listed above. For example, diethylstilbestrol (DES) which is known to cause vaginal cancer in humans, and bisphenol-A for which an estrogen (female) activity and toxicity have been observed are considered to have endocrine disrupting activities.

These substances are known to have the following primary activities: 1) direct activities on hormone receptors (e.g., synthetic hormone formulations, DDT, phthalate esters, etc.); 2) activities through other receptors (e.g., dioxins, etc.); 3) activities of inhibiting metabolism (e.g., steroid metabolic inhibitors, inhibitors of aromatase or 5 α-reductase, etc.); and 4) activities through other systems (e.g., substances that influence nervous system or immune system). Thus, their modes of actions are diverse [Kagaku (Chemistry), 53(7):12-15 (1998)].

As used herein, a gene that is influenced by an endocrine disruptor is defined as a gene of which the expression is promoted or suppressed by the above-listed endocrine disruptor as compared with a control. The number of the gene(s) may be one, or two or more. Thus, a gene for or related to an agent of which the expression is directly and/or indirectly influenced by an endocrine disruptor may be selected as the gene to be immobilized onto the DNA array of the present invention. Genes of which the expression is promoted and genes of which the expression is suppressed can be preferably used. Examples of preferable candidates for such genes (i.e., genes that are potentially influenced by endocrine disruptors) include, but are not limited to, those shown in Table 2 which are classified as follows: (1) a gene for a nuclear receptor or a gene related to nuclear receptor transcriptional coupling (nuclear receptor or nuclear receptor transcriptional coupling); (2) a gene related to kinase-type signal transduction (kinase-type signal transduction); (3) a gene related to gonad differentiation (gonad differentiation); (4) a gene for or related to a receptor-type kinase (receptor-type kinase); (5) a gene for or related to an intermediate filament marker (intermediate filament markers); (6) a gene related to cell cycle or growth regulation (cell cycle & growth regulators); (7) an oncogene, a gene related to an oncogene or a gene related to tumor suppression (oncogenes & tumor suppressors); (8) a gene related to apoptosis (apoptosis); (9) a gene related to damage response, repair or recombination of DNA (DNA damage response, repair & recombination); (10) a gene for or related to a receptor (receptors); (11) a gene related to cell death or differentiation regulation (cell fate & development regulators); (12) a gene related to adhesion, motility or invasion of cell (cell adhesion, motility & invasion); (13) a gene related to angiogenesis promotion (angiogenesis regulators); (14) a gene related to cellular invasion (invasion regulators); (15) a gene related to cell-cell interaction (cell-cell interactions); (16) a gene for or related to a Rho family, GTPase or a regulator therefor (Rho family small GTPases & their regulator); and (17) a gene for or related to a growth factor or a cytokine (growth factors & cytokines). Furthermore, other genes for or related to agents of which the expression is potentially influenced by endocrine disruptors in a direct and/or indirect manner are also included in the genes that are influenced by endocrine disruptors of the present invention.

**Table 2**

| **Gene Name** | **GenBank Accession #(s)** | **Classification** |
|---|---|---|
| type I cytoskeletal 10 keratin; cytokeratin 10 (K10) | X14487 | intermediate filament markers |
| cell division control protein 2 homolog (EC 2.7.1.-);cyclin-dependent kinase 1 (CDK1) | X05360 | cell cycle & growth regulators |
| cell division protein kinase 4 (EC 2.7.1.-) (PSK-J3) | M14505 | cell cycle & growth regulators |
| type I cytoskeletal 13 keratin; cytokeratin 13 (K13; CK 13) | X14640 | intermediate filament markers |
| type I cytoskeletal 14 keratin; cytokeratin 14 (K14; CK 14) | J00124 | intermediate filament markers |
| type I cytoskeletal 18 keratin; cytokeratin 18 (K18) | M26326 | intermediate filament markers |
| type I cytoskeletal 19 keratin; cytokeratin 19 (K19; CK 19) | Y00503 | intermediate filament markers |
| cyclin-dependent kinase 5 activator precursor (CDK5 activator) | X80343 | cell cycle & growth regulators |
| cell division cycle protein 25A tyrosine phosphatase (cdc25A); M-phase inducer phosphatase 1 (EC 3.1.3.48) | M81933 | cell cycle & growth regulators |
| CDC25B; M-phase inducer phosphatase 2 (EC 3.1.3.48) | S78187 | cell cycle & growth regulators |
| cdc25C; M-phase inducer phosphatase 3 (EC 3.1.3.48) | M34065 | cell cycle & growth regulators |
| CLK-2 | L29218 | cell cycle & growth regulators |
| CLK-3 | L29220 | cell cycle & growth regulators |
| serine/threonine-protein kinase KKIALRE | X66358 | cell cycle & growth regulators |
| type II cytoskeletal 11 keratin; cytokeratin 1 (K1; CK 1); 67-kDa cytokeratin; hair alpha protein | M98776 | intermediate filament markers |
| CDC2-related protein kinase CHED | M80629 | cell cycle & growth regulators |
| cdc2-related protein kinase PISSLRE | L33264 | cell cycle & growth regulators |
| cyclin A | X51688 | cell cycle & growth regulators |
| type II cytoskeletal 4 keratin; cytokeratin 4 (K4; CK4) | X07695 | intermediate filament markers |
| cyclin C G1/S-specific | M74091 | cell cycle & growth regulators |
| type II cytoskeletal 5 keratin; cytokeratin 5 (K5; CK 5); 58-kDa cytokeratin | M21389 | intermediate filament markers |
| cyclin D2 | D13639 | cell cycle & growth regulators |
| cyclin E | M73812 | cell cycle & growth regulators |
| cyclin G1 | X77794 | cell cycle & growth regulators |
| cyclin G2 | U47414 | cell cycle & growth regulators |
| type II cytoskeletal 6 keratin: cytokeratin 6B (CK 6B); K6B keratin | L42610 | intermediate filament markers |
| AMP deaminase isoform L (AMPD2) mRNA | M91029 | cell cycle & growth regulators |
| type II cytoskeletal 7 keratin; cytokeratin 7 (K7; CK 7) | M13955 | intermediate filament markers |
| CDK6 inhibitor 2c (p18) mRNA, complete cds | U17074 | cell cycle & growth regulators |
| type II cytoskeletal 8 keratin; cytokeratin 8 (K8; CK 8) | X74929 | intermediate filament markers |
| p35 cyclin-like CAK1-associated protein | X87843 | cell cycle & growth regulators |
| wee1Hu CDK tyrosine 15-kinase; wee-1-like protein kinase | U10564 | cell cycle & growth regulators |
| serine/threonine-protein kinase PLK (EC 2.7.1.-) (PLK-1) (STPK13) | U01038 | cell cycle & growth regulators |
| phospholipase D1 (PLD 1); choline phosphatase 1 | U38545 | cell cycle & growth regulators |
| CDC10 protein homolog | S72008 | cell cycle & growth regulators |
| CDC27HS Protein | S78234 | cell cycle & growth regulators |
| CDC16HS | U18291 | cell cycle & growth regulators |
| CDC37 homolog | U43077 | cell cycle & growth regulators |
| CDC6-related protein | U77949 | cell cycle & growth regulators |
| extracellular signal-regulated kinase 1 (ERK1) | X60188 | cell cycle & growth regulators |
| extracellular signal-regulated kinase 2 (ERK2) | Z11695 | cell cycle & growth regulators |
| extracellular signal-regulated kinase 3 (ERK3) | X80692 | cell cycle & growth regulators |
| extracellular signal-regulated kinase 4 (ERK4) | X59727 | cell cycle & growth regulators |
| extracellular signal-regulated kinase 5 (ERK5) | U29726 | cell cycle & growth regulators |
| mitogen-activated protein kinase p38 (MAP KINASE p38) | L35263 | cell cycle & growth regulators |
| vimentin | Z19554 | intermediate filament markers |
| profilin | J03191 | intermediate filament markers |
| c-jun N-terminal kinase 3 (JNK3) | U34819 | cell cycle & growth regulators |
| dual specificity mitogen-activated protein kinase kinase 5 | U25265 | cell cycle & growth regulators |
| dual-specificity mitogen-activated protein kinase kinase 1 | L11284 | cell cycle & growth regulators |
| dual-specificity mitogen-activated protein kinase kinase 6 | U39065 | cell cycle & growth regulators |
| PCNA; cyclin | M15796 | cell cycle & growth regulators |
| retinoblastoma-binding protein (RBP) | S66427 | cell cycle & growth regulators |
| RBQ1 retinoplastoma binding protein | X85133 | oncogenes & tumor suppressors |
| E2F-3 | D38550 | cell cycle & growth regulators |
| E2F-5 | U31556 | cell cycle & growth regulators |
| E2F-related transcription factor | L23959 | cell cycle & growth regulators |
| basic transcription factor 2 p44 (btf2p44) gene | U80017 | cell cycle & growth regulators |
| transcription factor DP2 (Humdp2); E2F dimerization partner 2 | U18422 | cell cycle & growth regulators |
| growth factor receptor-bound protein 2 (GRB2) isoform | M96995 | cell cycle & growth regulators |
| GRB-IR / GRB10 | D86962 | cell cycle & growth regulators |
| raf proto-oncogene serine/threonine-protein kinase (raf-1; c-raf) | X03484 | cell cycle & growth regulators |
| b-raf | M95712 | cell cycle & growth regulators |
| jun B transactivator | U20734 | cell cycle & growth regulators |
| N-myc oncogene protein | Y00664 | cell cycle & growth regulators |
| C-myc binding protein | D89667 | cell cycle & growth regulators |
| p53-dependent cell growth regulator CGR19 | U66469 | apoptosis |
| apoptosis regulator bcl-2 | M14745 | apoptosis |
| Bcl2 and p53 binding protein Bbp/53BP2 (BBP/53BP2) | U09582 | apoptosis |
| clone 53BP1 p53-binding protein mRNA, partial cds | U09477 | apoptosis |
| apoptosis regulator bcl-w; KIAA0271 | D87461 | apoptosis |
| induced myeloid leukemia cell differentiation protein MCL-1 | L08246 | apoptosis |
| bcl-2-related protein A1; bfl-1 protein | U29680 | apoptosis |
| BCL-2 homologous antagonist/killer (BAK) protein | U23765 | apoptosis |
| brain-related apoptosis gene (BRAG-1); Bcl-2 homolog | AB011170 | apoptosis |
| BAD protein (BCL-2 binding component 6) | U66879 | apoptosis |
| BCL2/adenovirus E1B 19kD-interacting protein 2 (BNIP2) mRNA, complete cds | U15173 | apoptosis |
| BCL2/adenovirus E1B 19kD-interacting protein 1 (BNIP1) mRNA, complete cds | AF083957 | apoptosis |
| interleukin-1 beta convertase precursor (IL-1 BC) | M87507 | apoptosis |
| apopain precursor; cysteine protease CPP32; YAMA protein | U13737 | apoptosis |
| ICH-2 protease precursor (EC 3.4.22.); TX protease (ICEREL-II); caspase-4 | U28014; U28015 | apoptosis |
| cysteine protease MCH2 isoforms alpha and beta (MCH2); caspase-6 precursor (EC 3.4.22.-) | U20537 | apoptosis |
| caspase-7 precursor (EC 3.4.22.-) | U37448 | apoptosis |
| Apo-2 ligand; TNF-related apoptosis inducing ligand TRAIL | U37518 | apoptosis |
| caspase-8 precursor (EC 3.4.22.-) | X98173 | apoptosis |
| caspase-9 precursor (EC 3.4.22.-) | U56390 | apoptosis |
| caspase-10 precursor; ICE-LIKE apoptotic protease 4 | U60519 | apoptosis |
| tyrosine-protein kinase receptor tyro3 precursor; tyrosine-protein kinase | D17517 | oncogenes & tumor suppressors |
| TRAF5 | AB000509 | apoptosis |
| TRAF6 | U78798 | apoptosis |
| TRAF-interacting protein I-TRAF; TRAF family member-associated NF-kB activator TANK | U59863 | apoptosis |
| TRAF-interacting protein (TRIP) | U77845 | apoptosis |
| serine/threonine protein kinase NIK; binds specifically to TRAF2 | Y10256 | apoptosis |
| casper, a FADD- and caspase-related inducer of apoptosis (CASH-alpha + CASH-beta) | AF015450 | apoptosis |
| cytotoxic ligand TRAIL receptor | AF016266 | apoptosis |
| death domain containing protein CRADD | U79115 | apoptosis |
| receptor interacting protein | U25994 | apoptosis |
| DAXX; a FAS-binding protein that activates JNK and apoptosis | AF039136 | apoptosis |
| tumor necrosis factor type 2 receptor associated protein (TRAP3) | U12597 | apoptosis |
| CD40 receptor associated factor 1 (CRAF1) | U21092 | apoptosis |
| inhibitor of apoptosis protein1 (HIAP1) (C-IAP2) | U45878 | apoptosis |
| inhibitor of apoptosis protein 2 (IAP-2) | U45879 | apoptosis |
| TNF-alpha converting enzyme | U69611 | apoptosis |
| ionizing radiation resistance-conferring protein; death-associated protein 3 (DAP-3) | U18321 | apoptosis |
| Fas-activated serine/threonine (FAST) kinase | X86779 | apoptosis |
| c-yes-1 | M15990 | oncogenes & tumor suppressors |
| FAS/APO 1 | D49396 | apoptosis |
| 5'-AMP activated protein kinase, gamma1 | U42412 | oncogenes & tumor suppressors |
| Akt1; rac protein kinase alpha; protein kinase B; c-Akt | M63167 | apoptosis |
| AKT2; rac protein kinase beta | M77198 | apoptosis |
| seven in absentia homolog | U63295 | apoptosis |
| signal transducer and activator of transcription 1-alpha/beta (STAT1) | M97935 | oncogenes & tumor suppressors |
| apoptosis-related protein TFAR15 (TFAR15) | AF022385 | apoptosis |
| signal transducer and transcription activator 5B (STAT5B) | U47686 | oncogenes & tumor suppressors |
| CD27BP (Siva) | U82938 | apoptosis |
| CSE1 | AF053640 | apoptosis |
| apoptosis inhibitor survivin | U75285 | apoptosis |
| proto-oncogene rhoA multidrug resistance protein; GTP-binding protein (rhoA) | L25080 | apoptosis |
| Pig7(PIG7) | AF010312 | apoptosis |
| Pig10 (PIG10) | AF010314 | apoptosis |
| Pig11 (PIG11) | AF010315 | apoptosis |
| Pig12 (PIG12) | AF010316 | apoptosis |
| glutathione-S-transferase homolog | U90313 | apoptosis |
| cdc42 homolog (G25K) (brain isoform + placental isoform) | U02570 | apoptosis |
| macrophage colony stimulating factor I receptor precursor (CSF-1-R) | X03663 | oncogenes & tumor suppressors |
| C-fos | V01512 | oncogenes & tumor suppressors |
| c-kit | X06182 | oncogenes & tumor suppressors |
| fgr proto-oncogene encoded p55-c-fgr protein | M19722 | oncogenes & tumor suppressors |
| DNA mismatch repair protein MSH2 | U03911 | oncogenes & tumor suppressors |
| DNA mismatch repair protein MSH6 (mutS alpha 160-kDa subunit) | U54777 | oncogenes & tumor suppressors |
| K-ras oncogene | M54968 | oncogenes & tumor suppressors |
| MET | J02958 | oncogenes & tumor suppressors |
| breast cancer susceptibility (BRCA2) | X95152 | oncogenes & tumor suppressors |
| BRCA1-associated ring domain protein | U76638 | oncogenes & tumor suppressors |
| p53 cellular tumor antigen | X54156 | oncogenes & tumor suppressors |
| mdm2 protein; p53-associated protein | M92424 | oncogenes & tumor suppressors |
| retinoblastoma susceptibility | L41870 | oncogenes & tumor suppressors |
| RB2/p130 | X74594 | oncogenes & tumor suppressors |
| RBA/p48 | X74262 | oncogenes & tumor suppressors |
| RBP2 retinoblastoma binding protein | S66431 | oncogenes & tumor suppressors |
| GADD153=growth arrest and DNA-damage-inducible | S40706 | DNA damage response, repair & recombination |
| insulin-like growth factor I receptor (IGF1R) | X04434 | receptors |
| DNA-PK catalytic subunit (XRCC7) | U47077 | DNA damage response, repair & recombination |
| ataxia telangiectasia (ATM) | U82828 | DNA damage response, repair & recombination |
| cation-independent mannose-6-phosphate receptor precursor (CI man-6-P receptor; CI-MPR) | Y00285 | receptors |
| Ku protein subunit; ATP-dependent DNA helicase II 70-kDa subunit | M32865 | DNA damage response, repair & recombination |
| Ku (p70/p80) subunit; ATP-dependent DNA helicase II 86-kDa subunit | M30938 | DNA damage response, repair & recombination |
| DNA excision repair protein ERCC1 | M13194 | DNA damage response, repair & recombination |
| DNA ligase III (LIG3); polydeoxyribonucleotide synthase | X84740 | DNA damage response, repair & recombination |
| DNA ligase IV; polydeoxyribonucleotide synthase (ATP) | X83441 | DNA damage response, repair & recombination |
| DNA polymerase alpha-subunit | X06745 | DNA damage response, repair & recombination |
| insulin-like growth factor binding protein 2 (IGFBP2) | X16302 | receptors |
| recA-like protein HsRad51; DNA repair protein RAD51 homolog | L07493 | DNA damage response, repair & recombination |
| DNA damage repair and recombination protein RAD52 | U12134 | DNA damage response, repair & recombination |
| DNA topoisomerase I (TOP1) | M60706 | DNA damage response, repair & recombination |
| growth hormone-dependent insulin-like growth factor-binding protein | M35878 | receptors |
| IGFBP5 | L27560 | receptors |
| DNA excision repair protein ERCC2 3' end; DNA-repair protein complementing XP-D cells | X52222 | DNA damage response, repair & recombination |
| IGFBP6 | M62402 | receptors |
| ERCC5 excision repair protein | X69978 | DNA damage response, repair & recombination |
| 6-O-methylguanine-DNA methyltransferase (MGMT); methylated-DNA-protein-cysteine methyltransferase | M29971 | DNA damage response, repair & recombination |
| muscle-specific DNase I-like (DNase X) | X90392 | DNA damage response, repair & recombination |
| DNA mismatch repair protein hmlh1 | U07418 | DNA damage response, repair & recombination |
| GTP-binding protein ras associated with diabetes (RAD1) | L24564 | DNA damage response, repair & recombination |
| replication, factor C 37-kDa subunit | M87339 | DNA damage response, repair & recombination |
| replication factor C 38-kDa subunit (RFC38); activator 1 38-kDa subunit | L07541 | DNA damage response, repair & recombination |
| replication protein A 70-kDa subunit (RP-A) (RF-A); single-stranded DNA-binding protein | M63488 | DNA damage response, repair & recombination |
| superoxide dismutase 1 cytosolic | X02317 | DNA damage response, repair & recombination |
| single-stranded DNA-binding protein pur-alpha | M96684 | DNA damage response, repair & recombination |
| HHR6A (yeast RAD 6 homolog) | M74524 | DNA damage response, repair & recombination |
| lysozyme | M19045 | DNA damage response, repair & recombination |
| Notch2 Notch homolog 3 | U97669 | cell fate & development regulators |
| CDW40 antigen; CD40L receptor precursor | X60592 | receptors |
| Jagged 1 | AF028593 | cell fate & development regulators |
| Jagged 2 | AF029778 | cell fate & development regulators |
| delta-like protein precursor (dlk); contains fetal antigen 1 (FA1) (DLK) | U15979 | cell fate & development regulators |
| lunatic fringe | U94354 | cell fate & development regulators |
| wnt-2 protein precursor; IRP protein; int-1 related protein | X07876 | cell fate & development regulators |
| Wnt-5a | L20861 | cell fate & development regulators |
| frizzled-related FrzB (Fritz); frezzled (fre) | U24163 | cell fate & development regulators |
| dishevelled 2 (DVL) | AF006012 | cell fate & development regulators |
| patched homolog (PTC) | U43148 | cell fate & development regulators |
| smoothened | U84401 | cell fate & development regulators |
| retinoic acid receptor epsilon (RAR-epsilon); retinoic acid receptor beta2 (RAR-beta2) | Y00291 | receptors |
| tumor necrosis factor type 1 receptor associated protein (TRAP1) mRNA, partial cds | U12595 | receptors |
| Tumor necrosis factor receptor 2 (75kD) (TNFR2) | U52165 | receptors |
| epidermal growth factor receptor substrate 15 (EPS15); AF-1P protein | U07707 | receptors |
| epidermal growth factor receptor kinase substrate EPS8 | U12535 | receptors |
| erythropoietin receptor (EPOR) | M60459 | receptors |
| NT-3 growth factor receptor precursor; trk-c tyrosine kinase; GP145-TRKC | U05012 | receptors |
| GARP | Z24680 | receptors |
| retinoic acid receptor alpha (RXRA) | X52773 | receptors |
| HGF activator like | D49742 | receptors |
| N-sam; fibroblast growth factor receptor1 precursor (FGFR1) | X66945 | receptors |
| low-affinity nerve growth factor receptor (NGF receptor; NGFR); GP80-LNGFR | M14764 | receptors |
| platelet-derived growth factor receptor alpha (PDGFRA); CD140A antigen | M21574 | receptors |
| beta platelet-derived growth factor receptor precursor (PDGFR-beta); CD140B antigen | J03278 | receptors |
| colon carcinoma kinase-4 (CCK4); transmembrane receptor precursor (PTK7) | U33635 | receptors |
| retinoic acid receptor gamma | M38258 | receptors |
| transforming growth factor (TGF)-beta receptor type III precursor (TGFR-3); betaglycan | L07594 | receptors |
| transmembrane protein TMP21 | AJ004913 | receptors |
| high-affinity nerve growth factor receptor precursor | X03541 | receptors |
| brain-derived neurotrophic factor (BDNF)/NT-3 growth factors receptor precursor | U12140 | receptors |
| hemopoietic progenitor cell CD34 antigen precursor | S53910 | cell adhesion, motility & invasion |
| CD59 | M84349 | cell adhesion, motility & invasion |
| angiopoietin 1 receptor precursor; tyrosine-protein kinase receptor TIE-2 | L06139 | angiogenesis regulators |
| collagen type I | J03464 | cell adhesion, motility & invasion |
| collagen type II alpha-1 | X16468 | cell adhesion, motility & invasion |
| collagen type III pro-alpha-1 | X14420 | cell adhesion, motility & invasion |
| collagen type IV alpha | M26576 | cell adhesion, motility & invasion |
| collagen type VI alpha-3 | X52022 | cell adhesion, motility & invasion |
| collagen type VIII alpha-1 | X57527 | cell adhesion, motility & invasion |
| vascular endothelial growth factor B precursor (VEGF-B) | U43368 | angiogenesis regulators |
| collagen type XI pro-alpha-2 | U32169 | cell adhesion, motility & invasion |
| collagen type XVI alpha-1 | M92642 | cell adhesion, motility & invasion |
| collagen type XVIII alpha | L22548 | cell adhesion, motility & invasion |
| laminin alpha-4 subunit precursor (LAMA4) | S78569 | cell adhesion, motility & invasion |
| laminin beta-2 subunit precursor (LAMB2); S-laminin | M94362 | cell adhesion, motility & invasion |
| laminin beta-1 subunit precursor (LAMB1); laminin B1 chain | M61916 | cell adhesion, motility & invasion |
| laminin gamma-1 subunit precursor (LAMG1); laminin B2 chain | M55210 | cell adhesion, motility & invasion |
| laminin 67kDa RECEPTOR | X15005 | cell adhesion, motility & invasion |
| nidogen precursor (NID); entactin | M30269 | cell adhesion, motility & invasion |
| tenascin precursor (TN); hexabrachion; cytotactin; neuronectin | X78565 | cell adhesion, motility & invasion |
| versican core protein precursor; large fibroblast proteoglycan | J02814 | cell adhesion, motility & invasion |
| sparc precursor (secreted protein acidic and rich in cysteine; osteonectin) (ON) | J03040 | cell adhesion, motility & invasion |
| thrombospondin 1 precursor | X14787 | cell adhesion, motility & invasion |
| thrombospondin 2 precursor | L12350 | cell adhesion, motility & invasion |
| vitronectin precursor; serum spreading factor; S-protein (contains somatomedin B) | X03168 | cell adhesion, motility & invasion |
| fibronectin precursor (FN) | X02761 | cell adhesion, motility & invasion |
| heparan sulfate proteoglycan (HSPG2) | M85289 | cell adhesion, motility & invasion |
| integrin alpha subunit | X68742 | cell adhesion, motility & invasion |
| vascular endothelial growth factor precursor (VEGF-C) | C U43142 | angiogenesis regulators |
| integrin alpha-3 chain | M59911 | cell adhesion, motility & invasion |
| integrin alpha-4 subunit precursor (integrin alpha-IV; ITGA4); VLA-4; CD49D antigen | L12002 | cell adhesion, motility & invasion |
| placenta growth factors 1 (PLGF-1) | X54936 | angiogenesis regulators |
| integrin alpha 7B | X74295 | cell adhesion, motility & invasion |
| integrin alpha9 | D25303 | cell adhesion, motility & invasion |
| integrin alpha-E precursor (ITGAE); mucosal lymphocyte-1 antigen; hml-1 antigen; CD103 antigen | L25851 | cell adhesion, motility & invasion |
| integrin beta1 | M34189 | cell adhesion, motility & invasion |
| integrin beta 4 | X53587 | cell adhesion, motility & invasion |
| integrin beta-5 subunit (ITGB5) | J05633 | cell adhesion, motility & invasion |
| integrin beta8 | M73780 | cell adhesion, motility & invasion |
| focal adhesion kinase (FADK); proline-rich tyrosine kinase 2 (PYK2) | L13616 | cell adhesion, motility & invasion |
| integrin-linked kinase (ILK) | U40282 | cell adhesion, motility & invasion |
| cell adhesion kinase beta (CAKbeta); protein tyrosine kinase Pyk2 | U43522 | cell adhesion, motility & invasion |
| paxillin | U14588 | cell adhesion, motility & invasion |
| alpha 1,2-mannosidase 1B mRNA | AF027156 | cell adhesion, motility & invasion |
| zyxin related protein ZRP-1 | AF000974 | cell adhesion, motility & invasion |
| beta 3-endonexin | U37139 | cell adhesion, motility & invasion |
| cytohesin-1; Sec7p-like protein | U70728 | cell adhesion, motility & invasion |
| CD9 antigen; p24; leukocyte antigen MIC3; motility-related protein (MRP-1) | M38690 | cell adhesion, motility & invasion |
| ezrin (cytovillin 2) | X51521 | cell adhesion, motility & invasion |
| moesin-ezrin-redixin-like protein; merlin; schwannomin; neurofibromatosis 2 | L11353 | cell adhesion, motility & invasion |
| neural cell adhesion molecule L1 precursor (N-CAM L1); MIC5 | AF002246 | cell adhesion, motility & invasion |
| ninjurin-1 | U91512 | cell adhesion, motility & invasion |
| formyl peptide receptor 1 | M60626 | cell adhesion, motility & invasion |
| P37NB | U32907 | cell adhesion, motility & invasion |
| semaphorin (CD100) | U60800 | cell adhesion, motility & invasion |
| semaphorin E | AB000220 | cell adhesion, motility & invasion |
| TAXI; axonin-1/TAQ1 | X67734 | cell adhesion, motility & invasion |
| leukocyte antigen related protein precursor (LAR); PTPRF | Y00815 | cell adhesion, motility & invasion |
| hyaluronan receptor (RHAMM) | U29343 | cell adhesion, motility & invasion |
| platelet glycoprotein IV (GPIV) (GPIIIB; CD36 antigen) (PAS IV); PAS-4 protein | M98399 | cell adhesion, motility & invasion |
| caveolin-2 | AF035752 | cell adhesion, motility & invasion |
| FGFR3; FLG-2 | M64347 | angiogenesis regulators |
| keratinocyte growth factor receptor (KGFR); fibroblast growth factor receptor 2 (FGFR2) | M80634 | angiogenesis regulators |
| MMP-1; collagenase-1 | X54925 | invasion regulators |
| MMP-2; gelatinase A | Z48482 | invasion regulators |
| MMP-16 | D85511 | invasion regulators |
| MMP-7; matrilysin | X07819 | invasion regulators |
| EB1 (protein that binds to APC) | U51677 | cell -cell interactions |
| MMP-10; stromelysin-2 | X07820 | invasion regulators |
| MMP-13; collagenase-3 | X75308 | invasion regulators |
| protocadherin 43 | L11373 | cell -cell interactions |
| desmoplakin I | M77830 | cell -cell interactions |
| envoplakin (EVPL) | U53786 | cell -cell interactions |
| bullous pemphigoid antigen | M69225 | cell-cell interactions |
| TIMP-2 (MI) | J05593 | invasion regulators |
| TIMP-3; mitogen-inducible gene 5 (mig-5) | Z30183 | invasion regulators |
| basigin precursor (BSG); leukocyte activation antigen M6 | X64364 | invasion regulators |
| urokinase-type plasminogen activator precursor (EC 3.4.21.73); U-plasminogen activator (UPA) | X02419 | invasion regulators |
| tissue-type plasminogen activator precursor (EC 3.4.21.68); T-plasminogen activator (T-PA) | M15518 | invasion regulators |
| plasminogen precursor (EC 3.4.21.7) | M34276 | invasion regulators |
| placental plasminogen activator inhibitor-2 (PAI-2); monocyte ARG-serpin; urokinase inhibitor; PLANH2 | Y00630 | invasion regulators |
| protein C inhibitor; plasma serine protease inhibitor precursor; plasminogen activator inhibitor-3 (PAI3) | M68516 | invasion regulators |
| urokinase-type plasminogen activator receptor | U09937 | invasion regulators |
| low-density lipoprotein receptor-related protein 1 precursor (LRP); alpha-2-macroglobulin receptor (A2MR) | X13916 | invasion regulators |
| alpha-2-macroglobulin precursor (alpha-2-M) | M11313 | invasion regulators |
| platelet basic protein precursor (PBP) | M54995 | invasion regulators |
| alpha-2-macroglobulin receptor-associated protein precursor (alpha-2-MRAP) | M63959 | invasion regulators |
| nucleoside diphosphate kinase A (EC 2.7.4.6) (NDK A) | X17620 | invasion regulators |
| c-myc purine-binding transcription factor puf, nucleoside diphosphate kinase B (NDP kinase B; NDK B) | M36981 | invasion regulators |
| nm23-H4; nucleoside-diphosphate kinase (EC 2.7.4.6); nucleoside 5'-diphosphate phosphotransferase (NDK) | Y07604 | invasion regulators |
| malignant melanoma metastasis-suppressor (KiSS-1) gene | U43527 | invasion regulators |
| metastasis-associated MTA1 | U35113 | invasion regulators |
| metalloprotease/disintegrin/cysteine-rich protein precursor (MDC9) | U41766 | invasion regulators |
| DDX8; RNA helicase | D50487 | invasion regulators |
| forkhead-like 7 | AF048693 | Rho family small GTPases & their regulator |
| rhoG | X61587 | Rho family small GTPases & their regulator |
| Rho6 protein | Y07923 | Rho family small GTPases & their regulator |
| Rho8 protein | X95282 | Rho family small GTPases & their regulator |
| ephrin-B3 precursor; eph-related receptor tyrosine kinase ligand 8 (LERK-8) | U66406 | cell -cell interactions |
| ras-like protein TC10 | M31470 | Rho family small GTPases & their regulator |
| ras-like small GTPase TTF | Z35227 | Rho family small GTPases & their regulator |
| rhoHP1 | D85815 | Rho family small GTPases & their regulator |
| rho-associated coiled-coil containing protein kinase p160ROCK | U43195 | Rho family small GTPases & their regulator |
| CDC42 GTPase-activating protein | U02570 | Rho family small GTPases & their regulator |
| T-lymphoma invasion and metastasis inducing TIAM1 | U16296 | Rho family small GTPases & their regulator |
| rho/rac guanine nucleotide exchange factor (rho/rac GEF); faciogenital dysplasia protein (FGD1) | U64105 | Rho family small GTPases & their regulator |
| ephrin type-A receptor 2 precursor; epithelial cell kinase (ECK); tyrosine-protein kinase receptor ECK | M59371 | cell-cell interactions |
| rho GDP dissociation inihibitor 2 (rho GDI 2); LY-GDI | L20688 | Rho family small GTPases & their regulator |
| rho GDP dissociation inihibitor 1 (rho GDI 1) | X69550 | Rho family small GTPases & their regulator |
| p21-activated kinase; p65-PAK; serine/threonine-protein kinase PAK-alpha | U24152 | Rho family small GTPases & their regulator |
| neural-cadherin precursor (N-cadherin); cadherin-2 | S42303 | cell -cell interactions |
| cadherin-3 placental-cadherin precursor, P-cadherin | X63629 | cell -cell interactions |
| cadherin-5 vascular endothelial-cadherin precursor, VE-cadherin; 7B4 antigen; CD144 antigen | X79981 | cell -cell interactions |
| cadherin-6 | D31784 | cell -cell interactions |
| cadherin-8 | L34060 | cell -cell interactions |
| casein kinase II, alpha subunit | J02853 | cell -cell interactions |
| ephrin type-B receptor 2 precursor; tyrosine-protein kinase receptor EPH-3; DRT; HEK; ERK | L41939 | cell -cell interactions |
| cadherin-13 T-cadherin precursor (truncated-cadherin); H-cadherin; heart-cadherin | U59289 | cell-cell interactions |
| cadherin-14 muscle-cadherin precursor; M-cadherin; cadherin-14; cadherin-15 | U59325 | cell-cell interactions |
| alpha-catenin; cadherin-associated protein; alpha E-catenin | D13866 | cell-cell interactions |
| alpha-catenin related protein (catenin alpha-2) | M94151 | cell -cell interactions |
| beta-catenin | X87838 | cell -cell interactions |
| tyrosine-protein kinase HCK (EC 2.7.1.112); P59-HCK & P60-HCK; hemopoietic cell kinase | M16591 | cell-cell interactions |
| APC | M73548 | cell-cell interactions |
| Tumor necrosis factor member2 (TNF) | X02910 | growth factors & cytokines |
| amphiregulin (AR); colorectum cell-derived growth factor (CRDGF) | M30704 | growth factors & cytokines |
| brain-derived neurotrophic factor (BDNF) | M61176 | growth factors & cytokines |
| beta NGF | X52599 | growth factors & cytokines |
| clone pSK1 interferon gamma receptor accessory factor-1 (AF-1); interferon-gamma receptor beta chain | U05875 | growth factors & cytokines |
| BIGH3 | M77349 | growth factors & cytokines |
| bone morphogenetic protein 1 (BMP1) | U50330 | growth factors & cytokines |
| interferon-alpha/beta receptor alpha subunit precursor (IFN-alpha receptor; IFNAR) | J03171 | growth factors & cytokines |
| bone morphogenetic protein 3B | D49493 | growth factors & cytokines |
| bone morphogenetic protein 2B (BMP2B) | D30751 | growth factors & cytokines |
| bone morphogenetic protein 6 | M60315 | growth factors & cytokines |
| bone morphogenetic protein 7; osteogenic protein 1 | X51801 | growth factors & cytokines |
| bone morphogenetic protein 8; osteogenic protein 2 | M97016 | growth factors & cytokines |
| BPGF-1 | L42379 | growth factors & cytokines |
| connective tissue growth factor (CTGF) | M92934 | growth factors & cytokines |
| heparin-binding EGF-like growth factor (HBEGF); diphtheria toxin receptor (DTR) | M60278 | growth factors & cytokines |
| interferon-alpha/beta receptor beta subunit precursor (IFN-alpha-R) | L42243 | growth factors & cytokines |
| fibrillin 2 (congenital contractural arachnodactyly) | U03272 | growth factors & cytokines |
| FGF2; heparin-binding growth factor 2 precursor; prostatropin | J04513 | growth factors & cytokines |
| keratinocyte growth factor (KGF); fibroblast growth factor 7 (FGF-7) | M60828 | growth factors & cytokines |
| cytokine humig; interferon-gamma-induced monokine (MIG) | X72755 | growth factors & cytokines |
| glia maturation factor beta (GMF-beta) | M86492 | growth factors & cytokines |
| glial growth factor precursor (GGFHPP2); neuregulin; heregulin-beta1 | L12261 | growth factors & cytokines |
| transforming growth factor beta2 precursor (TGF-beta2; TGFB2) | M19154 | growth factors & cytokines |
| interferon-gamma induced protein precursor (gamma-IP10) | X02530 | growth factors & cytokines |
| transcription factor ETR103; early growth response protein 1 (EGR-1) (KROX24) | X52541 | growth factors & cytokines |
| hepatocyte growth factor-like protein; macrophage-stimulating protein (MSP) | L11924 | growth factors & cytokines |
| hepatoma-derived growth factor (HDGF) | D16431 | growth factors & cytokines |
| hepatocyte growth factor (HGF); scatter factor (SF); hepatopoeitin A | X16323 | growth factors & cytokines |
| interleukin-1 receptor antagonist protein precursor (IL-1 RA; IRAP) | U65590 | growth factors & cytokines |
| interleukin-1 alpha precursor (IL-1 alpha; IL1A); hematopoietin-1 | M28983 | growth factors & cytokines |
| interleukin-1 beta precursor (IL-1 ; IL1B); catabolin | K02770 | growth factors & cytokines |
| MADER | X70991 | growth factors & cytokines |
| interleukin-6 precursor (IL-6); B-cell stimulatory factor 2 (BSF-2) | X04430 | growth factors & cytokines |
| interleukin-15 (IL-15) | U14407 | growth factors & cytokines |
| interferon gamma precursor (IFN-gamma; IFNG); immune interferon | V00543 | growth factors & cytokines |
| leukocyte interferon-inducible peptide | X57351 | growth factors & cytokines |
| leukemia inhibitory factor precursor (LIF); differentiation-stimulating factor (D factor) | X13967 | growth factors & cytokines |
| PDGF associated protein | U41745 | growth factors & cytokines |
| platelet-derived growth factor A subunit precursor (PDGFA; PDGF-1) | X06374 | growth factors & cytokines |
| platelet-derived growth factor B subunit precursor (PDGFB; PDGF2); bacaplermin; c-sis | X02811 | growth factors & cytokines |
| stromal cell derived factor 1 precursor (SDF1); pre B-cell growth stimulating factor (PBSF) | L36033 | growth factors & cytokines |
| TGF-b superfamily receptor type I (ALK-1) (SRK3) | L17075 | growth factors & cytokines |
| transforming growth factor-beta (TGF-beta3) | 3X14885 | growth factors & cytokines |
| thrombopoietin precursor (THPO); megakaryocyte colony stimulating factor | L33410 | growth factors & cytokines |
| transforming growth factor-alpha (TGF-alpha; TGFA); EGF-like TGF (ETGF) | X70340 | growth factors & cytokines |
| interferon-stimulated transcription factor 3, gamma (48kD) | M87503 | growth factors & cytokines |
| ubiquitin | S79522 | housekeeping gene |
| phospholipase A2 | U03090 | housekeeping gene |
| adenine phosphoribosyltransferase (APRT) | Y00486 | housekeeping gene |
| tubulin alpha | L11645 | housekeeping gene |
| HLA class I histocompatibility antigen A-3 alpha chain (MHC) | D32129 | housekeeping gene |
| aortic-type smooth muscle alpha-actin gene, exon 9 | 3216M3 | housekeeping gene |
| ribosomal protein S5 | U14970 | housekeeping gene |
| p300/CBP | U47741 | nuclear receptor or nuclear receptor transcriptional coupling |
| SRC-1 | U40396 | nuclear receptor or nuclear receptor transcriptional coupling |
| N-CoR/SMRT | AF044209/ U37146 | nuclear receptor or nuclear receptor transcriptional coupling |
| ACTR | AF036892 | nuclear receptor or nuclear receptor transcriptional coupling |
| RIP140 | X84373 | nuclear receptor or nuclear receptor transcriptional coupling |
| TRIP1 | L38810 | nuclear receptor or nuclear receptor transcriptional coupling |
| TIF2 | X97674 X97674 | nuclear receptor or nuclear receptor transcriptional coupling |
| Smad3 | AB004924 | nuclear receptor or nuclear receptor transcriptional coupling |
| efp | D21205 | nuclear receptor or nuclear receptor transcriptional coupling |
| lactoferrin | X53961 | nuclear receptor or nuclear receptor transcriptional coupling |
| progesteron receptor | M15716 | nuclear receptor or nuclear receptor transcriptional coupling |
| cathepsin G | J04990 | nuclear receptor or nuclear receptor transcriptional coupling |
| pS2 protein | X52003 | nuclear receptor or nuclear receptor transcriptional coupling |
| prolactin | E02152 | nuclear receptor or nuclear receptor transcriptional coupling |
| ARA70 | L49399 | nuclear receptor or nuclear receptor transcriptional coupling |
| vitamin D receptor | J03258 | nuclear receptor or nuclear receptor transcriptional coupling |
| p38 | L35253 | kinase-type signal transduction |
| p38 gamma | U66243 | kinase-type signal transduction |
| JNK1 | L26318 | kinase-type signal transduction |
| JNK2 | U09759 | kinase-type signal transduction |
| JNK3 | AA992006 | kinase-type signal transduction |
| ERK1 | M76585 | kinase-type signal transduction |
| BMKa,b,g | U29725-U29727 | kinase-type signal transduction |
| DAX1 | U31929 | gonad differentiation |
| SOX9 | Z46629 | gonad differentiation |
| WT1 | X51630 | gonad differentiation |
| SRY | L10101 | gonad differentiation |
| Ad4BP/SF-1 | D84206-D84209 | gonad differentiation |
| EMX2 | X68880 | gonad differentiation |
| c-Fos | K00650/ M16287 | oncogenes & tumor suppressors |
| c-Myc | J00120/ K01908 | oncogenes & tumor suppressors |
| Bcl-2 | M13994-M13995 | oncogenes & tumor suppressors |
| Bax a,b,g | L22473-L22475 | oncogenes & tumor suppressors |
| Bax d | U19599 | oncogenes & tumor suppressors |
| Bcl-x | U72398 | oncogenes & tumor suppressors |
| NGF receptor | M14764 | receptor-type kinase receptor-type kinase |
| FGF receptor | M34641 | receptor-type kinase |
| VEGF receptor | AF016050 | receptor-type kinase |
| PDGF receptor | M21616 | receptor-type kinase |
| CSF1 receptor | M33208-M33210 | receptor-type kinase |
| EGF receptor | M29366 | receptor-type kinase |
| insulin receptor | M10051 | receptor-type kinase |

The genes that are potentially influenced by endocrine disruptors are further exemplified by the gene for estrogen receptor, which is known to bind diethylstilbestrol, bisphenol-A, 17 β-estradiol and the like, as well as genes involved in the signal transduction pathway for the estrogen receptor.

A gene that is influenced by an endocrine disruptor can be detected as follows.

As used herein, a DNA array refers to a support onto which a gene or a DNA fragment derived from the gene is immobilized and includes, for example, a so-called DNA chip. Any supports which can be used for hybridization may be used. A slide glass, a silicone chip, a nitrocellulose or nylon membrane or the like is usually used. For example, the gene or a DNA fragment thereof to be immobilized onto the support can be prepared as follows. A primer pair for PCR amplification which is optimal for the method of the present invention can be prepared based on a base sequence identified by a GenBank accession no. assigned to a gene to be immobilized or the product of the gene using a primer analysis/construction software such as Oligo™ Primer Analysis Software (Takara Shuzo). A PCR-amplified fragment of interest can be obtained by using the primer pair and a genomic DNA, a genomic DNA library or a cDNA library as a template according to a standard protocol attached to a commercially available PCR kit. The resulting DNA fragment can be purified using, for example, Microcon-100 (Takara Shuzo). The purified DNA fragment can be preferably used in the method of the present invention. Furthermore, a DNA array can be prepared by immobilizing the gene or a fragment thereof onto a support according to a known method, for example, by introducing an amino group to the support. Also, a DNA array onto which gene are arrayed and immobilized at high density can be prepared by conducting the immobilization procedure using an instrument for preparing DNA arrays such as an instrument for preparing DNA chips from GMS.

The use of such a DNA array makes it possible to simultaneously measure the contents of various nucleic acid molecules in a nucleic acid sample and has the advantage that the measurement can be conducted using a small amount of a nucleic acid sample.

Any genes or DNA fragments derived from the genes are immobilized onto the DNA array used in the present invention. Preferably, genes encoding proteins that are expected to have functions related to endocrine disrupting activities or DNA fragments derived from the genes are immobilized. If a fragment is used, a fragment of, for example, about 1 kb in length can be preferably used, although the length of the fragment is not limited to specific one. The length may be shorter or longer than that described above as long as the fragment specifically hybridizes with a nucleic acid from a test sample. Examples of such genes include, but are not limited to, a gene for a hormone receptor, a gene encoding a cofactor for a receptor, a gene encoding a protein involved in signal transduction from a receptor, a gene encoding a protein involved in biosynthesis or metabolism of a hormone and an oncogene.

For example, mRNAs prepared from a cell or a tissue (organ) that is sensitive to an endocrine disruptor or cDNAs obtained by reverse transcription using the mRNAs as templates can be used as nucleic acid samples containing a gene of which the expression is altered as a result of the influence of the endocrine disruptor. Such mRNAs are obtained over time or on different days after being exposed to the endocrine disruptor.

The cell to be exposed to a sample containing an endocrine disruptor may be a cell collected from an organism, or it may be a cultured cell. The tissue is not limited to specific one as long as it is supposed to be influenced by an endocrine disruptor. Furthermore, the origin of the cell or the tissue, or the organism to be used is not limited to human. The length of the time of exposure to an endocrine disruptor may vary depending on the organism, the endocrine disruptor, the gene that is influenced by the endocrine disruptor or the like to be used.

On the other hand, a nucleic acid sample containing mRNAs, or cDNAs therefor, similarly prepared from a cell, a tissue or an organism as a control is subjected to hybridization under stringent conditions. The nucleic acid sample can be suitably labeled as follows such that it can be readily determined whether or not the nucleic acid sample hybridizes with a DNA on a DNA array.

For example, a radioisotope, a fluorescent substance, chemiluminescent substance, an antigen recognized by an appropriate antibody or the like can be used for labeling. Alternatively, hybridization may be first conducted without labeling the nucleic acid sample, and then an intercalating substance that emits fluorescence or chemiluminescence may be used for labeling.

Hybridization of the thus obtained nucleic acid sample with the DNA on the DNA array can be conducted according to a known method. It is natural to conduct hybridization and washing steps under optimal conditions depending on the length of the DNA on the DNA array or the like. These steps can be conducted under conditions, for example, as described in Molecular Cloning, A Laboratory Manual, 2nd ed., pp. 9.52-9.55 (1989).

By comparing results of hybridization for a control nucleic acid sample with those for a nucleic acid sample derived from a cell, a tissue or an organism which has been exposed to a sample containing an endocrine disruptor, a gene of which the signal intensity is significantly different among the two nucleic acid samples can be detected. Specifically, an array is subjected to hybridization with a nucleic acid sample labeled as described above. A signal intensity of radioactivity, fluorescence, luminescence or the like for the hybridized array is detected using a specialized measuring instrument such as a chromatogram scanner or image analyzer. A gene of which the expression is significantly altered as a result of the influence of an endocrine disruptor can be detected based on the difference in the signal intensity.

The gene expression for a control nucleic acid sample can be compared on the same DNA array with that for a nucleic acid sample derived from a cell, a tissue or an organism that has been exposed to an endocrine disruptor on the same DNA array by using a multiple wavelength detecting fluorescence analyzer which is capable of detecting plural labels (e.g., two types of fluorescence). For example, a nucleic acid sample derived from a cell which has been exposed to an endocrine disruptor is fluorescence-labeled with Cy3-dUTP, whereas a control nucleic acid sample is fluorescence-labeled with Cy5-dUTP. The difference in gene expression between the two nucleic acid samples can be detected as difference in color by mixing equal amounts of the nucleic acid samples and subjecting the mixture to hybridization with a DNA array. A gene of which the expression level is significantly altered as a result of the influence of the endocrine disruptor can be detected based on the results.

The gene is also useful as an index for detecting an endocrine disruptor.

A gene that is influenced by an endocrine disruptor is selected by comparing a signal intensity detected as an index of expression level with that obtained using a nucleic acid sample prepared using a control sample. For example, a value is calculated by dividing a fluorescence signal value for a sample containing an endocrine disruptor by a fluorescence signal value for a control sample. A value greater than 1.00 indicates that the gene expression is promoted by the treatment with the test substance. A value smaller than 1.00 indicates that the gene expression is suppressed by the treatment with the test substance. A value equal to 1.00 indicates that the gene is not influenced by the treatment with the test substance. If the expression is promoted, the value is greater than 1.10, preferably 1.30, more preferably 2.00. If the expression is suppressed, the value is smaller than 0.90, preferably 0.80, more preferably 0.70.

As described above, the expression of genes that are influenced by endocrine disruptors (for example, a gene for a nuclear receptor in a cell and a number of genes involved in the downstream signal transduction pathway) can be detected simultaneously, in vitro, rapidly and with high sensitivity according to the method of the present invention. In addition, it is possible to find involvement of a known gene in a previously unknown signal transduction pathway.

(2) An endocrine disruptor can be detected using the expression of a gene that is influenced by the endocrine disruptor as an index as follows.

A DNA array, onto which a gene which has been confirmed to be influenced by an endocrine disruptor is immobilized as described above, is prepared.

A nucleic acid sample is prepared from a cell, a tissue or an organism which is suspected to be influenced by the endocrine disruptor as described above. The nucleic acid sample is then hybridized as described above. Change in gene expression can be determined based on the difference between the signal intensities. The presence of the endocrine disruptor can be determined based on the results.

In another embodiment, the presence of an endocrine disruptor can be also determined as follows. An RNA or a DNA that competes with an mRNA for a gene that has been confirmed to be influenced by the endocrine disruptor is prepared. A competitive RT-PCR is conducted using the RNA or the DNA as an internal standard. The expression level of the influenced gene is then quantitatively determined by the competitive RT-PCR.

As described above, the presence or the absence of an endocrine disruptor can be substantially and readily judged using the expression of a gene that is influenced by a endocrine disruptor (for example, a gene for a nuclear receptor in a cell or one of a number of downstream genes) as an index according to the method of the present invention.

(3) A substance that potentially causes endocrine disruption can be detected as follows.

As used herein, a substance that potentially causes endocrine disruption means a substance that potentially influences the normal activity of a hormone which is naturally exerted in a living body. Substances of which the activities have been already confirmed and substances of which the activities have not been confirmed yet are included within the definition.

A DNA array for detecting a substance that potentially causes endocrine disruption is prepared by immobilizing in a manner as described above a gene which has been confirmed to be influenced by an endocrine disruptor according to a method as described in (1) above.

A nucleic acid sample is prepared from a cell, a tissue or an organism which has been exposed to a sample that is suspected to contain a substance that potentially causes endocrine disruption as described above. The nucleic acid sample is then hybridized as described above. Change in gene expression can be determined based on the difference between the signal intensities. The substance can be judged as an endocrine disruptor based on the results.

A substance can be considered to be an endocrine disruptor based on the results not only in case where changes in signals are observed for all of the DNAs on the DNA array but also in case where the changes are observed for a portion of the DNAs. In particular, if the changes in signal strength are observed for a portion of the DNAs, the detection method can be optimized by further selecting the genes that are influenced by the substance action according to the method as described in (1) above such that a substance that causes endocrine disruption as the substance does can be detected more exactly.

In another embodiment, an RNA or a DNA that competes with an mRNA for a gene that has been confirmed to be influenced by the endocrine disruptor as described above is prepared. A competitive RT-PCR is conducted using the RNA or the DNA as an internal standard. The degree of endocrine disruption can be quantitatively detected based on the expression of the gene. Any methods can be preferably used for detecting, or quantifying the expression of, a gene that is influenced by an endocrine disruptor obtained as described in (1) above as long as the methods can be used for the detection/quantification of the gene.

### (4) The DNA array of the present invention

As used herein, a DNA array refers to a support onto which a gene or a fragment thereof is immobilized and includes, for example, a so-called DNA chip.

Any supports which can be used for hybridization may be used for the DNA array of the present invention. Usually, a slide glass, a silicone chip, a nitrocellulose or nylon membrane or the like is used. Preferably, a support made from a material which is non-porous and has a smooth surface (e.g., a glass such as a slide glass) can be preferably used. Any supports having surfaces onto which DNAs can be immobilized through covalent bonds or noncovalent bonds can be used. Supports having hydrophilic or hydrophobic functional groups on their surfaces are preferably used. Examples of the preferable functional groups on the surfaces of the supports include, but are not limited to, a hydroxy group, an amino group, a thiol group, an aldehyde group, a carboxyl group and an acyl group. The functional group may be present as a surface property of the support itself, or it may be introduced by surface treatment. Examples of supports with surface treatment include a glass treated with a commercially available silane coupling agent such as aminoalkylsilane or treated with a polycation such as polylysine or polyethyleneimine. Several treated slide glasses are commercially available.

A gene that is influenced by an endocrine disruptor or a DNA fragment derived from the gene is immobilized onto the DNA array of the present invention. The DNA array may be a DNA microarray in which double-stranded DNAs of the genes or the DNA fragments derived from the genes are arrayed and immobilized onto the same support under denaturing conditions. At least a portion of the immobilized DNA may be single-stranded. The DNA array herein may be prepared by spotting double-stranded DNAs onto the same support in array under denaturing conditions. The density of the array in the DNA array of the present invention is not specifically limited. For example, a DNA array with high density such as an array onto which DNAs are immobilized at 100 dot/cm² or more can be preferably used.

The DNA fragment to be arrayed and immobilized onto a support in the present invention is not limited to specific one. In general, a double-stranded polynucleotide of 50 bases or more in length or a derivative thereof, which is prepared by enzymatic amplification by polymerase chain reaction (PCR) or the like and converted into single-stranded DNAs or derivatives thereof by denaturing upon immobilization onto a support for immobilizing a DNA, can be preferably used. The derivative may have modification which enables the immobilization onto the surface of the support. Examples of the derivatives include, but are not limited to, a DNA into which a functional group such as an amino group or a thiol group is introduced at the 5'-terminus of the DNA.

For example, a DNA amplified by PCR or the like using a genomic DNA library or a cDNA library as a template can be used as a gene or a DNA fragment to be immobilized onto a support. An oligonucleotide synthesized based on a known nucleotide sequence can also be used. A nucleic acid other than a DNA which is known in the art to be able to be used for hybridization (for example, an RNA prepared by in vitro transcription) can be immobilized in place of a DNA. The DNA array can be prepared by immobilizing the gene or a fragment thereof onto a support according to a known method, for example, by introducing an amino group to the support. The DNA array of the present invention onto which genes are arrayed and immobilized can be prepared by conducting the immobilization procedure using an instrument for preparing DNA arrays such as an instrument for preparing DNA chips from GMS.

A gene encoding a protein having a function involved in the exertion of an activity of a hormone, or a fragment thereof, is immobilized onto the DNA array used in the present invention. If a fragment is used, a fragment of, for example, about 100 b to about 1 kb in length can be preferably used, although the length of the fragment is not limited to specific one. The length may be shorter or longer than that described above as long as the fragment specifically hybridizes with a nucleic acid from a test sample. Examples of such genes include, but are not limited to, a gene for a hormone receptor, a gene encoding a cofactor for a receptor, a gene encoding a protein related to signal transduction from a receptor, a gene encoding a protein related to biosynthesis or metabolism of a hormone, an oncogene and the like. In addition, a gene that is influenced by an endocrine disruptor, for example, obtained according to the method as described in (1) above may be immobilized. Furthermore, since all of the genes that are influenced by endocrine disruptors can be obtained according to the method as described in (1) above, a DNA array for detecting genes that are influenced by endocrine disruptors onto which all of such genes are immobilized can be prepared.

As described above, for example, a gene for a nuclear receptor in a cell and genes related to the downstream signal transduction pathway can be detected in vitro, rapidly and with high sensitivity by using the method and the DNA array of the present invention. Thus, the presence or the absence of a substance that potentially causes endocrine disruption can be substantially and readily judged.

The following examples further illustrate the present invention in detail but are not to be construed to limit the scope thereof.

### Example 1

Genes that are potentially influenced by endocrine disruptors are shown in Table 3.

**Table 3**

| Agent (Gene product) | GenBank accession no. |
|---|---|
| 1. Nuclear receptor or nuclear receptor transcriptional coupling | |
| p300/CBP | U47741 |
| SRC-1 | U40396 |
| N-CoR/SMRT | AF044209/U37146 |
| ACTR | AF036892 |
| RIP140 | X84373 |
| TRIP1 | L38810 |
| TIF2 | X97674 |
| Smad3 | AB004924 |
| efp | D21205 |
| lactoferrin | X53961 |
| progesteron receptor | M15716 |
| cathepsin G | J04990 |
| pS2 protein | X52003 |
| prolactin | E02152 |
| ARA70 | L49399 |
| vitamin D receptor | J03258 |
| 2. Kinase-type signal transduction | |
| p38 | L35253 |
| p38 gamma | U66243 |
| JNK1 | L26318 |
| JNK2 | U09759 |
| JNK3 | AA992006 |
| ERK1 | M76585 |
| BMK α, β, γ | U29725-U29727 |
| 3. Gonad differentiation | |
| DAX1 | U31929 |
| SOX9 | Z46629 |
| WT1 | X51630 |
| SRY | L10101 |
| Ad4BP/SF-1 | D84206-D84209 |
| EMX2 | X68880 |
| 4. Oncogenes | |
| c-Fos | K00650/M16287 |
| c-Myc | J00120/K01908 |
| Bcl-2 | M13994-M13995 |
| Bax α, β, γ | L22473-L22475 |
| Bax 5 | U19599 |
| Bcl-x | U72398 |
| 5. Receptor-type kinase | |
| NGF receptor | M14764 |
| FGF receptor | M34641 |
| VEGF receptor | AF016050 |
| PDGF receptor | M21616 |
| CSF1 receptor | M33208-M33210 |
| EGF receptor | M29366 |
| insulin receptor | M10051 |

Fragments of about 1 kb that contain the 3'-untranslated regions of cDNAs for these genes were prepared as follows.

Briefly, cDNA fragments of interest were amplified by reverse transcription PCR (RT-PCR) using mRNAs from cells or tissues derived from humans or mice (Clontech) as templates. The amplified cDNAs were confirmed to be the fragments of interest by analyzing their base sequences. The amplified fragments were recovered by ethanol precipitation and dissolved in 10 mM carbonate buffer (pH 9.5) at a concentration of 1 µM. In addition, β-actin gene as a housekeeping gene and a plasmid pUC18 as a negative control were similarly prepared. These fragments were spotted onto a slide glass with introduced amino groups (Sigma) using an instrument for preparing DNA chips (GMS) and fixed by UV irradiation. The slide glass was washed with 0.2% SDS followed by distilled water and dried to prepare a DNA array.

### Example 2

### (1) Administration into mouse

10 female mice (2 days old) were divided into a group with endocrine disruptor administration and a group without administration. Diethylstilbestrol (DES), which potentially causes endocrine disruption, was intravenously injected into each mouse in the group with administration at 0.1 mg/mouse/day for 2 days. Ovaries were removed on day 4, and mRNAs were prepared using an mRNA extraction kit (Qiagen).

cDNAs synthesis reactions were carried out using mixtures each containing about 3 µg of the mRNA, oligo-dT primer, Cy3-dUTP (Amersham) for the group with administration or Cy5-dUTP (Amersham) for the group without administration, dNTPs and reverse transcriptase (Gibco-BRL). The mixtures were subjected to gel filtration, concentrated under reduced pressure and dissolved in 4 x SSC/0.2% SDS to prepare fluorescence-labeled cDNAs.

### (2) Treatment of cultured cells

Human breast cancer MCF-7 cells were grown in DME medium containing 5% fetal bovine serum (FBS). After trypsinization, 2 x 10⁵ cells were placed in each well of a 12-well culture plate. The cells were incubated in the same medium for 24 hours. After the medium was removed, the cells were cultured for 72 hours in DME medium containing 5% human serum from which steroid hormones had been removed by treatment with activated carbon-dextran in the presence or absence of 17-β estradiol at a concentration of 10 pM. The cells were recovered, and mRNAs were extracted as described in Example 2-(1).

cDNAs synthesis reactions were carried out using mixtures each containing about 3 µg of the mRNA, oligo-dT primer, Cy3-dUTP for the cells exposed to 17-β estradiol or Cy5-dUTP for the cells not exposed to 17-β estradiol, dNTPs and reverse transcriptase (Gibco-BRL). The mixtures were subjected to gel filtration, concentrated under reduced pressure and dissolved in 4 x SSC/0.2% SDS to prepare fluorescence-labeled cDNAs.

### (3) Hybridization of labeled cDNA with DNA array

Equal volumes of the Cy3-labeled cDNA and the Cy5-labeled cDNA as prepared in (1) above were mixed together and heat-denatured. 5 µl of the mixture was dropped onto the DNA array as prepared in Example 1. A cover glass was placed on the mixture and the sides of the cover glass were sealed with a film. After incubation at 40-45°C for 10 hours, the cover glass was removed. The DNA array was washed in 0.2 x SSC/0.1% SDS for 30 minutes and in 0.2 x SSC for 30 minutes, and then air-dried. The fluorescent signals from the respective spots on the DNA array were analyzed using a microarray scanner (GMS). Furthermore, similar procedure was carried out for the labeled cDNAs obtained in (2) above.

As a result, significant changes in signals were observed for the ovary from the mouse administered with DES and MCF-7 cells exposed to 17-β estradiol. Thus, genes that were influenced by endocrine disruptors could be detected.

### Example 3

### (1) Preparation of DNA array

33 genes were selected from the genes listed in Table 3 in Example 1. The selected genes are shown in Table 4. β-Actin gene and a plasmid pBR322 were used as a housekeeping gene and as a negative control, respectively.

**Table 4**

| Gene no. | Immobilized gene (Gene product) | Primer pair (SEQ ID NO) |
|---|---|---|
| 1. | Smad3 | 1, 2 |
| 2. | VEGF receptor | 3, 4 |
| 3. | ACTR | 5, 6 |
| 4. | N-CoR/SMRT | 7, 8 |
| 5. | efp | 9, 10 |
| 6. | c-Myc-1 | 11, 12 |
| 7. | Vitamin D receptor | 13, 14 |
| 8. | cathepsin G | Commercially available |
| 9. | c-Myc-2 | 15, 16 |
| 10. | Bax | 17, 18 |
| 11. | JNK1 | 19, 20 |
| 12. | p38 | 21, 22 |
| 13. | TRIP 1 | 23, 24 |
| 14. | ARA 70 | 25, 26 |
| 15. | insulin receptor | 27, 28 |
| 16. | NGF receptor | Commercially available |
| 17. | PDGF receptor | 29, 30 |
| 18. | CSF1 receptor-1 | Commercially available |
| 19. | CSF1 receptor-2 | 31, 32 |
| 20. | FGF receptor | 33, 34 |
| 21. | p38 gamma | 35, 36 |
| 22. | Bcl-X | 37, 38 |
| 23. | c-Myc-3 | 39, 40 |
| 24. | pS2 protein | 41, 42 |
| 25. | lactoferrin | 43, 44 |
| 26. | RIP 140 | 45, 46 |
| 27. | TIF2 | 47, 48 |
| 28. | JNK2 | 49, 50 |
| 29. | Bax delta | 51, 52 |
| 30. | BMK-1 | 53, 54 |
| 31. | BMK-2 | 55, 56 |
| 32. | Src-1 | 57, 58 |
| 33. | p300/CBP | 59, 60 |
| 34. | β-actin (positive control) | 61, 62 |
| 35. | pBR 322 (negative control) | |

cDNA fragments for these genes of about 100 b to about 1 kb were prepared using the respective primer pairs as indicated in Table 4 according to the method as described in Example 1, and spotted to prepare a DNA array. Genes for cathepsin G, NGF receptor and CSF1 receptor were amplified using primers for the respective genes contained in Human UniGene DNA set (Research Genetics).

### (2) Examination of influence by endocrine disruptor

Influences by treatment with various endocrine disruptors for 2 or 24 hours on cultured cells were examined.

Treatment for 2 hours: Human breast cancer MCF-7 cells were grown in DME medium containing 10% fetal bovine serum (FBS). After trypsinization, 2 x 10⁶ cells were placed in a 10-cm dish. The cells were cultured for 24 hours in DME medium containing 5% fetal bovine serum from which steroid hormones had been removed by treatment with activated carbon-dextran. After removing the medium, the cells were cultured for 2 hour in the same medium containing 10 nM 17-β estradiol (E₂), 10 nM diethylstilbestrol (DES) or 5 µM bisphenol-A (BisA). As a control, cells which were cultured in the absence of such a chemical substance were similarly prepared. The treated cells were recovered, and total RNAs were extracted as described in Example 2(1).

Treatment for 24 hours: Human breast cancer MCF-7 cells were grown in DME medium containing 10% fetal bovine serum (FBS). After trypsinization, 2 x 10⁶ cells were placed in a 10-cm culture dish. The cells were incubated for 24 hours in the same medium. After removing the medium, the cells were cultured for 24 hours in DME medium containing 5% human serum from which steroid hormones had been removed by treatment with activated carbon-dextran in the presence of 10 nM 17-β estradiol (E₂), 10 nM diethylstilbestrol (DES) or 5 µM bisphenol-A (BisA). As a control, cells which were cultured in the absence of such a chemical substance were similarly prepared. The treated cells were recovered, and total RNAs were extracted as described in Example 2(1).

(3) The total RNAs as prepared in (2) above were treated with DNase. Reaction mixtures each containing about 100 to about 300 µg of the total RNA, 10 µl of 10 x AMV buffer (Life Science) and 10 U of DNaseI (Takara Shuzo) in a volume of 12 µl were prepared, incubated at 37°C for 10 minutes, extracted twice with phenol/chloroform, and then subjected to ethanol precipitation. The concentrations of the resulting total RNAs were determined using portions thereof.

(4) Reverse transcription reaction was carried out using one of the total RNAs as prepared in (3) above. The composition of the reaction mixture was as follows.

Reaction mixture A: about 130 µg of the total RNA, 10 µg of oligo-dT primer (Takara Shuzo) and 20 µl of water treated with diethylpyrocarbonate (DEPC, Wako Pure Chemical Industries).

Reaction mixture B: 12 µl of 5 x AMV RTase buffer (Life Science), 0.5 mM each of dATP, dCTP and dGTP, 0.2 mM dTTP, 60 U of RNase inhibitor (Takara Shuzo) and 0.1 mM Cy3-labeled dUTP (Amersham Pharmacia).

The reaction mixture A was incubated at 70°C for 10 minutes and then cooled on ice. The reaction mixture B was added thereto, and the resulting mixture was incubated at 42°C for 5 minutes. About 60 U of AMV RTase (Life Science) was added thereto. DEPC-treated water was further added to make the final volume to 60 µl. The resulting RT reaction mixture was incubated at 42°C for 70 minutes. 7.5 µl of 500 mM EDTA solution and 15 µl of 1 M sodium hydroxide were added to the reaction mixture. The mixture was incubated at 60°C for 1 hour to degrade the template RNA. After cooling to room temperature, 37.5 µl of 1 M tris-hydrochloride (pH 7.5) was added to the mixture. The solution was concentrated to 20 µl using Microcon YM-30 (Millipore), 200 µl of 10 mM tris-hydrochloride containing 1 mM EDTA was added thereto, and the resulting mixture was then concentrated to 20 µl. The thus obtained Cy3-labeled cDNA solution was used for the subsequent hybridization.

(5) The Cy3-labeled cDNA as prepared in (4) above was heat-denatured, and the whole solution was dropped onto the DNA array as prepared in (1) above. A cover glass was placed on the spotted solution and the sides of the cover glass were sealed with a film. After incubation at 40-45°C for 10 hours, the cover glass was removed. The DNA array was washed in 0.2 x SSC/0.1% SDS for 30 minutes and in 0.2 x SSC for 30 minutes, and then air-dried. The fluorescent signals from the respective spots on the DNA array were analyzed using a microarray scanner (GMS). Representative values each obtained by dividing a fluorescence signal value for a sample treated with one of the substances by a fluorescence signal value for a control sample are shown in Table 5. In Table 5, a value greater than 1.00 indicates that the gene expression is promoted by the treatment with the substance. A value smaller than 1.00 indicates that the gene expression is suppressed by the treatment with the substance. A value equal to 1.00 indicates that the gene is not influenced by the treatment with the test substance.

**Table 5**

| Immobilized gene (Gene product) | DES treatment 2hr / 24hr | BisA treatment 2hr / 24hr | E₂ treatment 2hr / 24hr |
|---|---|---|---|
| Nuclear receptor or nuclear receptor transcriptional coupling | | | |
| p300/CBP | 1.28 / 4.45 | 1.50 / 1.07 | 1.67 / 3.39 |
| N-CoR/SMRT | 0.62 / 1.42 | 1.17 / 1.08 | 1.86 / 1.51 |
| ACTR | 1.14 / 4.97 | 0.47 / 1.03 | 1.19 / 3.27 |
| RIP 140 | 1.74 / 2.51 | 1.70 / 1.14 | 1.46 / 2.34 |
| TIF2 | 1.19 / 3.04 | 2.66 / 0.84 | 2.17 / 3.30 |
| ARA 70 | 0.63 / 1.37 | 1.31 / 0.93 | 1.47 / 1.45 |
| | | | |
| Kinase-type signal transduction | | | |
| JNK2 | 0.72 / 1.85 | 1.30 / 0.71 | 1.86 / 1.71 |
| BMK-2 | 1.15 / 6.06 | 1.04 / 0.29 | 1.13 / 0.05 |
| | | | |
| Oncogenes | | | |
| c-Myc-1 | 1.08 / 0.00 | 1.40 / 1.30 | 2.52 / 1.89 |
| Bax | 1.34 / 2.27 | 2.41 / 1.44 | 1.32 / 0.99 |
| | | | |
| Receptor-type kinase | | | |
| PDGF receptor | 0.65 / 3.04 | 1.25 / 1.19 | 1.63 / 2.89 |
| VEGF receptor | 1.15 / 3.27 | 0.37 / 0.46 | 2.13 / 2.94 |

In many cases, abnormal reproduction in wild animals and reduced spermatogenesis in humans presumably caused by disruption by endocrine disrupting activities are considered to be due to suppression or interruption of signals for endocrine action at a certain stage. Therefore, it is considered that genes of which the expression is suppressed when compared with the expression in control cells should be noticed in addition to overexpressed genes. For example, among the genes used in this example, promotion of the expression of many genes that are considered to be closely related to the action of estrogen (e.g., genes for p300/CBP, ACTR, RIP 140, TIF2, PDGF receptor and VEGF receptor) by stimulation with E₂ was observed as shown in Table 5, although little is known about the pathway, the mechanism or the like. The treatment with DES, which causes endocrine disruption, for 24 hours activated almost all of the genes other than c-Myc. Suppression of the expression of the genes for N-CoR/SMRT and ARA 70 (nuclear receptor or nuclear receptor transcriptional coupling), p38 gamma (data not shown) and JNK2 (kinase-type signal transduction), and PDGF receptor (receptor-type kinase) was observed for. the treatment with DES for 2 hours. On the other hand, the treatment with BisA, which is suspected to have an endocrine disrupting activity, for 2 hours suppressed the expression of the genes for ACTR (nuclear receptor or nuclear receptor transcriptional coupling) and VEGF receptor (receptor-type kinase). The influence by the treatment with BisA on the genes was considered to be less than that observed for the stimulation with DES. The treatment with BisA for 24 hours suppressed the expression of the genes for JNK2 and BMK2 (kinase-type signal transduction), and VEGF receptor (receptor-type kinase). Thus, control of gene expression by BisA treatment in a manner different from that observed for the stimulation with DES was observed. As described above, the use of the chip of the present invention provides a method in which significant variation of signals for expression depending on the substances used for treatment and the length of treatment time is observed. A gene that is influenced by an endocrine disruptor, in particular, of which the expression is suppressed by an endocrine disruptor, could be clearly detected.

### Industrial Applicability

As described above, the method of the present invention is excellently effective in that it can detect a number of genes that are influenced by endocrine disruptors simultaneously, in vitro, rapidly and with high sensitivity. Furthermore, the present invention provides a DNA array which can be used to detect genes that are influenced by endocrine disruptors rapidly and with high sensitivity. The method of the present invention is also useful for detecting a gene involved in a previously unknown signal transduction pathway. In addition, the present invention is excellently effective in that the presence or the absence of an endocrine disruptor or a substance that potentially causes endocrine disruption can be judged using the expression of a number of genes that are influenced by endocrine disruptors obtained according to the method of the present invention as an index.

### Sequence Listing Free Text

SEQ ID NO:1: Designed oligonucleotide primer to amplify Smad3 mRNA.
SEQ ID NO:2: Designed oligonucleotide primer to amplify Smad3 mRNA.
SEQ ID NO:3: Designed oligonucleotide primer to amplify VEGF receptor mRNA.
SEQ ID NO:4: Designed oligonucleotide primer to amplify VEGF receptor mRNA.
SEQ ID NO:5: Designed oligonucleotide primer to amplify ACTR mRNA.
SEQ ID NO:6: Designed oligonucleotide primer to amplify ACTR mRNA.
SEQ ID NO:7: Designed oligonucleotide primer to amplify N-CoR/SMRT mRNA.
SEQ ID NO:8: Designed oligonucleotide primer to amplify N-CoR/SMRT mRNA.
SEQ ID NO:9: Designed oligonucleotide primer to amplify efp mRNA.
SEQ ID NO:10: Designed oligonucleotide primer to amplify efp mRNA.
SEQ ID NO:11: Designed oligonucleotide primer to amplify c-Myc-1 mRNA.
SEQ ID NO:12: Designed oligonucleotide primer to amplify c-Myc-1 mRNA.
SEQ ID NO:13: Designed oligonucleotide primer to amplify vitamin D receptor mRNA.
SEQ ID NO:14: Designed oligonucleotide primer to amplify vitamin D receptor mRNA.
SEQ ID NO:15: Designed oligonucleotide primer to amplify c-Myc-2 mRNA.
SEQ ID NO:16: Designed oligonucleotide primer to amplify c-Myc-2 mRNA.
SEQ ID NO:17: Designed oligonucleotide primer to amplify Bax mRNA.
SEQ ID NO:18: Designed oligonucleotide primer to amplify Bax mRNA.
SEQ ID NO:19: Designed oligonucleotide primer to amplify JNK1 mRNA.
SEQ ID NO:20: Designed oligonucleotide primer to amplify JNK1 mRNA.
SEQ ID NO:21: Designed oligonucleotide primer to amplify p38 mRNA.
SEQ ID NO:22: Designed oligonucleotide primer to amplify p38 mRNA.
SEQ ID NO:23: Designed oligonucleotide primer to amplify TRIP 1 mRNA.
SEQ ID NO:24: Designed oligonucleotide primer to amplify TRIP 1 mRNA.
SEQ ID NO:25: Designed oligonucleotide primer to amplify ARA 70 mRNA.
SEQ ID NO:26: Designed oligonucleotide primer to amplify ARA 70 mRNA.
SEQ ID NO:27: Designed oligonucleotide primer to amplify insulin receptor mRNA.
SEQ ID NO:28: Designed oligonucleotide primer to amplify insulin receptor mRNA.
SEQ ID NO:29: Designed oligonucleotide primer to amplify PDGF receptor mRNA.
SEQ ID NO:30: Designed oligonucleotide primer to amplify PDGF receptor mRNA.
SEQ ID NO:31: Designed oligonucleotide primer to amplify CSF1 receptor-2 mRNA.
SEQ ID NO:32: Designed oligonucleotide primer to amplify CSF1 receptor-2 mRNA.
SEQ ID NO:33: Designed oligonucleotide primer to amplify FGF receptor mRNA.
SEQ ID NO:34: Designed oligonucleotide primer to amplify FGF receptor mRNA.
SEQ ID NO:35: Designed oligonucleotide primer to amplify p38 gamma mRNA.
SEQ ID NO:36: Designed oligonucleotide primer to amplify p38 gamma mRNA.
SEQ ID NO:37: Designed oligonucleotide primer to amplify Bcl-X mRNA.
SEQ ID NO:38: Designed oligonucleotide primer to amplify Bcl-X mRNA.
SEQ ID NO:39: Designed oligonucleotide primer to amplify c-Myc-3 mRNA.
SEQ ID NO:40: Designed oligonucleotide primer to amplify c-Myc-3 mRNA.
SEQ ID NO:41: Designed oligonucleotide primer to amplify pS2 protein mRNA.
SEQ ID NO:42: Designed oligonucleotide primer to amplify pS2 protein mRNA.
SEQ ID NO:43: Designed oligonucleotide primer to amplify lactoferrin mRNA.
SEQ ID NO:44: Designed oligonucleotide primer to amplify lactoferrin mRNA.
SEQ ID NO:45: Designed oligonucleotide primer to amplify RIP 140 mRNA.
SEQ ID NO:46: Designed oligonucleotide primer to amplify RIP 140 mRNA.
SEQ ID NO:47: Designed oligonucleotide primer to amplify TIF2 mRNA.
SEQ ID NO:48: Designed oligonucleotide primer to amplify TIF2 mRNA.
SEQ ID NO:49: Designed oligonucleotide primer to amplify JNK2 mRNA.
SEQ ID NO:50: Designed oligonucleotide primer to amplify JNK2 mRNA.
SEQ ID NO:51: Designed oligonucleotide primer to amplify Bax delta mRNA.
SEQ ID NO:52: Designed oligonucleotide primer to amplify Bax delta mRNA.
SEQ ID NO:53: Designed oligonucleotide primer to amplify BMK-1 mRNA.
SEQ ID NO:54: Designed oligonucleotide primer to amplify BMK-1 mRNA.
SEQ ID NO:55: Designed oligonucleotide primer to amplify BMK-2 mRNA.
SEQ ID NO:56: Designed oligonucleotide primer to amplify BMK-2 mRNA.
SEQ ID NO:57: Designed oligonucleotide primer to amplify Src-1 mRNA.
SEQ ID NO:58: Designed oligonucleotide primer to amplify Src-1 mRNA.
SEQ ID NO:59: Designed oligonucleotide primer to amplify p300/CBP mRNA.
SEQ ID NO:60: Designed oligonucleotide primer to amplify p300/CBP mRNA.
SEQ ID NO:61: Designed oligonucleotide primer to amplify β-actin mRNA.
SEQ ID NO:62: Designed oligonucleotide primer to amplify β-actin mRNA.

## Claims

1. A method for detecting a gene that is influenced by an endocrine disruptor, characterized in which the method comprises:
preparing a nucleic acid sample containing mRNAs, or cDNAs therefor, derived from a cell, a tissue or an organism which has been exposed to a sample containing an endocrine disruptor;
hybridizing the nucleic acid sample with a DNA array onto which genes which are potentially influenced by the endocrine disruptor or DNA fragments derived from the genes which are potentially influenced by the endocrine disruptor are immobilized; and
selecting a gene that is influenced by the endocrine disruptor by comparing the results with results for a nucleic acid sample prepared using a control sample.

2. The method according to claim 1, wherein a gene selected from the group consisting of:
(1) a gene for a nuclear receptor or a gene related to nuclear receptor transcriptional coupling;
(2) a gene related to kinase-type signal transduction;
(3) a gene related to gonad differentiation;
(4) a gene for or related to a receptor-type kinase;
(5) a gene for or related to an intermediate filament marker;
(6) a gene related to cell cycle or growth regulation;
(7) an oncogene, a gene related to an oncogene or a gene related to tumor suppression;
(8) a gene related to apoptosis;
(9) a gene related to damage response, repair or recombination of DNA;
(10) a gene for or related to a receptor;
(11) a gene related to cell death or differentiation regulation;
(12) a gene related to adhesion, motility or invasion of cell;
(13) a gene related to angiogenesis promotion;
(14) a gene related to cellular invasion;
(15) a gene related to cell-cell interaction;
(16) a gene for or related to a Rho family, GTPase or a regulator therefor; and
(17) a gene for or related to a growth factor or a cytokine,
or a DNA fragment derived from the gene is used.

3. A method for detecting an endocrine disruptor, characterized in which the method comprises measuring the expression of the gene detected by the method according to claim 1 or 2.

4. The method according to claim 3, wherein the endocrine disruptor is selected from ones classified into:
(1) dioxins;
(2) organochlorine compounds;
(3) phenols;
(4) phthalate esters;
(5) aromatic hydrocarbons;
(6) pesticides;
(7) organotin compounds;
(8) estrogens; or
(9) mirex, toxaphene, aldicarb or kepone.

5. A method for detecting a substance that potentially causes endocrine disruption, characterized in which the method comprises:
preparing a nucleic acid sample containing mRNAs, or cDNAs therefor, derived from a cell, a tissue or an organism which has been exposed to a sample that is suspected to contain a substance that potentially causes endocrine disruption;
hybridizing the nucleic acid sample with a DNA array onto which genes which are influenced by an endocrine disruptor or DNA fragments derived from the genes which are influenced by the endocrine disruptor are immobilized; and
detecting a substance that potentially causes endocrine disruption by comparing the results with results for a nucleic acid sample prepared using a control sample.

6. The method according to claim 5, wherein the substance that potentially causes endocrine disruption is classified into:
(1) dioxins;
(2) organochlorine compounds;
(3) phenols;
(4) phthalate esters;
(5) aromatic hydrocarbons;
(6) pesticides;
(7) organotin compounds;
(8) estrogens; or
(9) mirex, toxaphene, aldicarb or kepone.

7. A DNA array for detecting a gene that is influenced by an endocrine disruptor, onto which a gene that is influenced by an endocrine disruptor or a gene that is potentially influenced by an endocrine disruptor, or a DNA fragment derived from the gene is immobilized.

8. The DNA array according to claim 7, onto which a gene selected from the group consisting of:
(1) a gene for a nuclear receptor or a gene related to nuclear receptor transcriptional coupling;
(2) a gene related to kinase-type signal transduction;
(3) a gene related to gonad differentiation;
(4) a gene for or related to a receptor-type kinase;
(5) a gene for or related to an intermediate filament marker;
(6) a gene related to cell cycle or growth regulation;
(7) an oncogene, a gene related to an oncogene or a gene related to tumor suppression;
(8) a gene related to apoptosis;
(9) a gene related, to damage response, repair or recombination of DNA;
(10) a gene for or related to a receptor;
(11) a gene related to cell death or differentiation regulation;
(12) a gene related to adhesion, motility or invasion of cell;
(13) a gene related to angiogenesis promotion;
(14) a gene related to cellular invasion;
(15) a gene related to cell-cell interaction;
(16) a gene for or related to a Rho family, GTPase or a regulator therefor; and
(17) a gene for or related to a growth factor or a cytokine,
or a DNA fragment derived from the gene is immobilized.

9. The DNA array according to claim 7 or 8, wherein the gene or the DNA fragment derived from the gene is immobilized onto a slide glass.
